# EUROPÄISCHE PATENTSCHRIFT

(11) **EP 0 731 918 B1**
(45) Veröffentlichungstag und Bekanntmachung des Hinweises auf die Patenterteilung: **03.09.1997**
(21) Anmeldenummer: 95903779.7
(22) Anmeldetag: 29.11.1994
(51) Int. Cl.: G01N 33/68, A61B 5/14, A61J 1/05

(54) **VERFAHREN ZUR STABILISIERUNG VON OSTEOCALCIN IN HUMANEN SERUMS- ODER PLASMAPROBEN FÜR DIE BESTIMMUNG DES OSTEOCALCINGEHALTS SOWIE INSTRUMENTE UND PROBENGEFÄSSE ZUR DURCHFÜHRUNG DIESES VERFAHRENS**
PROCESS FOR STABILISING OSTEOCALCIN IN HUMAN SERUM OR PLASMA SAMPLES FOR THE PURPOSE OF DETERMINING OSTEOCALCIN CONTENT, AND INSTRUMENTS AND SAMPLE VESSELS FOR THE SAID PROCESS
PROCEDE DE STABILISATION DE L'OSTEOCALCINE DANS DES ECHANTILLONS DE SERUM ET DE PLASMA HUMAINS POUR DETERMINER LA TENEUR EN OSTEOCALCINE, INSTRUMENTS ET RECIPIENTS POUR ECHANTILLONS PERMETTANT LA MISE EN OEUVRE DUDIT PROCEDE

(30) Priorität: 29.11.1993 DE 4340597
(43) Veröffentlichungstag der Anmeldung: 18.09.1996
(73) Patentinhaber: B.R.A.H.M.S. DIAGNOSTICA GmbH, 12099 Berlin (DE)
(72) Erfinder: BERGMANN, Andreas, D-12351 Berlin (DE); WECKERMANN, Renate, D-10555 Berlin (DE)
(74) Vertreter: Andrae, Steffen, Dr.
(86) Internationale Anmeldenummer: EP9403962
(87) Internationale Veröffentlichungsnummer: WO9514933

(56) Entgegenhaltungen:
- EP-A- 0 557 663
- DE-C- 3 833 936
- J. BONE MINER. RES. (1990), 5(5), 451-61 CODEN: JBMREJ;ISSN: 0884-0431, 1990 TRACY, R. P. ET AL 'Comparison of monoclonal and polyclonal antibody-based immunoassays for osteocalcin: a study of sources of variation in assay results'
- EUROPEAN JOURNAL OF CANCER & CLINICAL ONCOLOGY, Bd.24, Nr.7, Juli 1988, OXFORD, GB Seiten 1211 - 1217 R.E. COLEMAN, G. MASHITER, I. FOGELMAN, K.D. WHITAKER, M. CALEFFI, D.W. MOSS AND R.D. RUBENS 'Osteocalcin: a Potential Marker of Metastatic Bone Disease and Response to Treatment' in der Anmeldung erwähnt
- CLINICAL CHEMISTRY 40 (11 PART 1). 1994. 2071-2077. ISSN: 0009-9147 DIAZ DIEGO E M ET AL 'Six Osteocalcin Assays Compared.'
- CLIN. CHEM. (WASHINGTON, D. C.) (1994), 40(5), 833-4 CODEN: CLCHAU;ISSN: 0009-9147, 1994 BANFI, GIUSEPPE ET AL 'In vitro stability of osteocalcin'

## Beschreibung

Die Erfindung betrifft ein Verfahren zur Verhinderung des Abbaus von Osteocalcin in humanem Serum oder Plasma sowie Instrumente und Probengefäße zur Durchführung dieses Verfahrens. Das erfindungsgemäße Verfahren kann auch als Verfahren zur Stabilisierung von endogenem Osteocalcin in humanen Serum- oder Plasmaproben bezeichnet werden, da es darauf beruht, daß der während der Zeit zwischen der Probengewinnung und der Messung des Osteocalcins normalerweise zu beobachtende Abbau des endogenen Osteocalcins verhindert wird.

Humanes Osteocalcin, das auch als Vitamin K-abhängiges Knochenprotein oder γ-Carboxyglutaminsäure enthaltendes Protein [im englischsprachigen Schrifttum: bone Gla protein (BGP)] bezeichnet wird, ist ein spezifischer Peptidbestandteil der Knochenmatrix. Das Peptid besteht aus 49 Aminosäuren und weist die folgende Aminosäuresequenz auf:

Tyr-Leu-Tyr-Gln-Trp-Leu-Gly-Ala-Pro-Val-Pro-Try-Pro-Asp-Pro-
Leu-Glu-Pro-Arg-Arg-Gla-Val-Cys-Gla-Leu-Asn-Pro-Asp-Cys-Asp-
Glu-Leu-Ala-Asp-Arg-Ile-Gly-Phe-Gln-Glu-Ala-Tyr-Arg-Arg-Phe-
Tyr-Gly-Pro-Val49

Das Peptid wird von den Osteoblasten synthetisiert, und ein geringer Teil des gebildeten Osteocalcins gelangt in das Blut. Über die Bestimmung der Konzentration dieses Osteocalcins im Blut sind bestimmte wertvolle Rückschlüsse auf den Stoffwechsel des Knochens möglich, wobei zur ergänzenden Information auf die Arbeit von Lian, J.B. und Gundberg, C.M. in: Clinical Orthopaedics and Related Research, 226, Seite 267 bis 291 (1988) verwiesen wird. Über die Bestimmung der Osteocalcinkonzentration in humanem Serum oder Plasma können danach möglicherweise hilfreiche Informationen für die Diagnose und Therapie von Knochenstoffwechselstörungen erhalten werden.

Zur Messung der Osteocalcinkonzentration wurden verschiedene Verfahren entwickelt. So ist nach an sich bekannten Verfahren die Bestimmung der Osteocalcinkonzentration auf immundiagnostischem Wege möglich, beispielsweise nach dem Verfahren gemäß US-A-4 438 208 und der in dem Patent DE 38 33 936 der Anmelderin beschriebenen verbesserten Ausführungsform eines solchen Verfahrens.

Die EP 0 557 663 A1 betrifft ein immundiagnostisches Bestimmungsverfahren, bei dem ein ganz spezieller monoklonaler Antikörper gegen Osteocalcin verwendet wird, der bestimmte Konformationen erkennt, die für das sogenannte decarboxylierte oder untercarboxylierte Osteocalcin typisch sind. Das decarboxylierte oder untercarboxylierte Osteocalcin unterscheidet sich dadurch von carboxyliertem Osteocalcin, daß bei ersterem die ursprünglichen GLU-Reste in den Positionen 17, 21 und 24 des Osteocalcinmoleküls nicht oder nicht vollständig zu GLA-Resten carboxyliert sind. Der unterschiedliche Carboxylierungsgrad führt dazu, daß sich die Formen konformativ unterscheiden.

Damit man mit einem "konformativen" Antikörper der verwendeten Art auch die Gesamtmenge an Osteocalcin messen kann, d.h. also auch das carboxylierte Osteocalcin erfassen kann, wird der Probe für die Bestimmung ein Calciumsalz zugesetzt, da sich gezeigt hat, daß sich in Anwesenheit des Calciumsalzes die Konformation des carboxylierten Osteocalcins so verändert, daß der spezielle Antikörper auch das carboxylierte Osteocalcin erkennt und dieses für Meßzwecke genau wie das de- bzw. untercarboxylierte Osteocalcin bindet. Es geht in der EP 0 557 663 Al nicht um eine Stabilisierung des Osteocalcins zwischen der Probennahme und der eigentlichen immundiagnostischen Bestimmung.

Unabhängig davon, nach welchem Verfahren die Osteocalcinkonzentration bestimmt wird, ist es für eine zuverlässige und klinisch relevante Bestimmung des Osteocalcins in der Probe Voraussetzung, daß die gemessene Osteocalcinkonzentration die Konzentration zum Zeitpunkt der Blutabnahme wiederspiegelt. Konzentrationsveränderungen im Zeitraum zwischen Blutentnahme und eigentlicher Konzentrationsmessung führen nämlich dazu, daß unbrauchbare Meßergebnisse erhalten werden.

Es ist nunmehr bereits bekannt, daß endogenes Osteocalcin in Blutproben abgebaut wird (siehe Lian und Gundberg (1988) a. a. O. und Coleman et al (1988) Eur. J. Cancer Clin. Oncol. 24, 1211-1217). Das hat zur Folge, daß in Abhängigkeit von der Art und Dauer der Lagerung der Probe vor der Messung "falsch niedrige" Osteocalcinkonzentrationen im Serum oder Plasma gefunden werden. Es wurde bereits vorgeschlagen, dieses Problem dadurch zu umgehen, daß man Seren/Plasmaproben unmittelbar nach ihrer Gewinnung vor einer Lagerung einfriert oder lyophilisiert. Beide Verfahren sind jedoch unpraktisch (z. B. der Transport von Seren in gefrorenem Zustand) oder teuer und aufwendig (Lyophilisation).

Eine sofortige Messung der Osteocalcinkonzentration unmittelbar im Anschluß an eine Probennahme ist nur in den seltensten Fällen möglich, keines-falls jedoch in der normalen ärztlichen Praxis, wo es üblich ist, die von den Patienten gewonnenen Proben zur Untersuchung an ein Labor zu verschicken.

Es ist somit Aufgabe der vorliegenden Erfindung, ein verbessertes Verfahren zur Bestimmung von Osteocalcin in humanem Serum oder Plasma zu schaffen, bei dem der Abbau des endogenen Osteocalcins in der Probe verhindert und damit als Fehlerquelle beseitigt werden kann und bei dem zur Probenstabilisation keine aufwendigen, unüblichen technischen Maßnahmen getroffen werden müssen.

Diese Aufgabe wird bei einem Verfahren gemäß Oberbegriff von Anspruch 1 dadurch gelöst, daß man der Probe unmittelbar bzw. während ihrer Gewinnung eine zur Verhinderung des Osteocalcinabbaus ausreichende Menge an divalenten Metallionen, insbesondere an Calciumionen, zusetzt.

Vorteilhafte Ausgestaltungen des erfindungsgemäßen Verfahrens sind in den Unteransprüchen 2 bis 6 beschrieben.

Zur Durchführung des erfindungsgemäßen Verfahrens können besonders präparierte Instrumente oder Probengefäße verwendet werden, wie sie in den Ansprüchen 7 und 8 näher beschrieben sind.

Bei der Schaffung der vorliegenden Erfindung wurde der Weg eingeschlagen, eine Substanz zu suchen, die, dem Serum oder Plasma zugesetzt, den Abbau des endogenen Osteocalcins verhindert.

Dabei wurde von der auch von Lian und Grundberg (10c. cit.) geteilten Annahme ausgegangen, daß der Abbau des Osteocalcins proteolytischer Natur ist, d. h. von im Serum und Plasma vorhandenen Proteasen verursacht wird. Daher wurde zuerst versucht, den Osteocalcinabbau durch Zusatz geeigneter Proteolysehemmstoffe zu unterdrücken, beispielsweise solcher, wie sie in dem Verfahren gemäß Patent DE 38 33 936 erfolgreich zur Verhinderung des Tracerabbaus eingesetzt wurden. Es zeigte sich, daß es nicht möglich ist, mit den bisher erprobten Proteolysehemmstoffen den Abbau des Osteocalcins zu verringern. Als dann versucht wurde, die ebenfalls als Proteolysehemmstoffe bekannten Metallchelatoren EDTA und o-Phenanthrolin zur Hemmung des Osteocalcinabbaus zuzusetzen, wurde überraschenderweise statt einer Hemmung eine Beschleunigung der Osteocalcinhydrolyse beobachtet.

Aus dieser Beobachtung wurden dann Schlüsse gezogen, die die Schaffung des erfindungsgemäßen Verfahrens ermöglichten: Wenn die beschleunigende Wirkung der Metallchelatoren auf den Osteocalcinabbau darauf beruht, daß die Metallionenkonzentration in der Probe verringert wird, ist diese Metallionenkonzentration für die Abbaubarkeit des Osteocalcins wichtig. Es wurde daher versucht, durch eine Erhöhung der Metallionenkonzentration im Serum eine Stabilisierung des Osteocalcins zu erhalten.

Es zeigte sich, daß der Zusatz von löslichen Calciumsalzen, z. B. von Calciumchlorid CaCl₂, den Abbau von Osteocalcin völlig unterdrückt, wenn die zugesetzten Konzentrationen richtig gewählt werden. Zu diesem Zwecke wurde eine Reihenuntersuchung durchgeführt, die ergab, daß erst oberhalb von 20 mM CaCl₂ in der Probe keine Veränderung der Osteocalcinkonzentration, nachgewiesen anhand der Osteocalcin-Immunreaktivität, mehr erhalten wurde.

Offensichtlich wird durch Zusatz von Calciumionen und anderen, wie Calciumionen wirkenden zweiwertigen Metallionen das Osteocalcin in einen Zustand überführt, in dem es durch die anwesenden Proteasen nicht mehr abbaubar ist, ohne gleichzeitig die für seine Bestimmung auf immundiagnostischem Wege wesentliche Immunreaktivität zu verlieren.

Als Metallionen, die gleich oder ähnlich wie Calciumionen wirken, kommen insbesondere zweiwertige Metallionen und dabei insbesondere wieder Magnesiumionen, Zinkionen, Cobaltionen in Betracht, es ist jedoch vorstellbar, daß sich in Kenntnis des erfindungsgemäßen Verfahrens mittels einfacher Reihenversuche auch noch weitere zweiwertige oder möglicherweise sogar dreiwertige Metallionen finden lassen, die im Sinne des erfindungsgemäßen Verfahrens zur Stabilisierung der Osteocalcinkonzentration wirksam sind.

Es ist ferner denkbar, daß gewisse Substanzen, von denen bekannt ist, daß sie biochemisch ähnlich wirken wie Calciumionen, erfolgreich zur Verhinderung des Osteocalcinabbaus eingesetzt werden können.

Die Metallionen sollen der Probe aus einleuchtenden Gründen grundsätzlich so früh wie möglich nach der Probengewinnung zugesetzt werden, um das Einsetzen eines Osteocalcinabbaus in der gwonnenen Probe von Anfang an zu unterbinden.

Die Metallionen können grundsätzlich in beliebiger Form zugesetzt werden, solange sie ausreichend in der Probe in Lösung gehen. So können sie in Form löslicher fester Salze oder in Form von Lösungen in wäßrigen oder wassermischbaren Lösungsmitteln zugesetzt werden, solange die verwendeten Lösungsmittel, die nachfolgende Messung nicht stören.

In der Praxis kann das erfindungsgemäße Verfahren in verschiedener Weise vorteilhaft verwirklicht werden. So können Kits zur Osteocalcinbestimmung Substanzen und/oder präparierte Probengefäße beigefügt werden. Beispielsweise können die erforderlichen Calciumkonzentrationen in Form von Calciumtabletten beigefügt werden.

Da die Stabilisierung jedoch bereits bei der Probengewinnung zu erfolgen hat, während die eigentliche Messung an einem ganz anderen Ort vorgenommen wird, ist es für die in der täglichen ärztlichen Praxis durchzuführenden Probennahmen von Vorteil, wenn spezielle Instrumente oder Probengefäße für die Osteocalcinbestimmung dem Arzt direkt zur Verfügung gestellt werden. So können dem Arzt Calciumtabletten in der richtigen Dosierungsmenge für die Probenstabilisation zur Verfügung gestellt werden, es können ihm jedoch auch speziell präparierte Instrumente, wie beispielsweise Blutentnahmespritzen oder spezielle Gefäße für die Aufbewahrung und die Verschickung der Proben zur Verfügung gestellt werden, die die erforderlichen Metallkonzentrationen in geeigneter Form von vorn herein enthalten. Eine vorteilhaft erscheinende Möglichkeit der Präparierung derartiger Instrumente oder Probengefäße besteht darin, auf der Wand einen festen, in der Probe löslichen Film anzubringen, der die Metallionen enthält und an die Probe freigibt. Dieser Film kann ausschließlich ein aufgetrockneter Salzfilm sein, er kann aber auch ein neutral reagierendes, probenlösliches Bindemittel enthalten. Die Metallionen werden in Form geeigneter wasserlöslicher Salze mit verträglichen Anionen eingesetzt. So lassen sich die bevorzugten Calciumionen erfolgreich in Form von Calciumchlorid einführen. Schlechtlösliche oder unlösliche Salze scheiden als ungeeignet in der Regel aus, genau wie solche Salze, deren Anionen für die Stabilität der Probe oder die nachfolgende Messung schädlich sind, beispielsweise wegen einer unerwünschten Beeinflussung des pH-Wertes.

Nachfolgend wird das erfindungsgemäße Verfahren anhand von Versuchen und Vergleichsversuchen noch näher erläutert.

Es zeigen:
- **Fig. 1**: die Abnahme der Osteocalcinkonzentration in vier Serumproben als prozentuelle Abnahme der Osteocalcin-Immunreaktivität in einem RIA;
- **Fig. 2**: den Effekt der Zugabe von CaCl₂ (20 mM) zu Proben entsprechend Fig. 1; und
- **Fig. 3**: den Effekt der Zugabe von CaCl₂ (20 mM) zu Proben, bei denen die Osteocalcinkonzentration als prozentuale verbleibende Osteocalcin-Immunreaktivität in einem anderen kommerziellen RIA als in den Fig. 1 und 2 bestimmt wurde.
- **Fig. 4**: den Vergleich des Osteocalcinabbaus in Serumproben mit und ohne CaCl₂ (20 mM) bei 4-tägiger Lagerung bei Raumtemperatur

### Versuchsbeschreibungen

Der Effekt verschiedener Substanzen und Zusatzmengen auf die Serum- und Plasmaproben im Hinblick auf die Stabilisierung der Osteocalinkonzentration in den Proben wurde bestimmt, indem die Osteocalcinmengen in den Proben zu verschiedenen Zeitpunkten und unter verschiedenen Bedingungen mit Hilfe des OSCAtest® Osteocalcin BGP (Henning Berlin GmbH) ermittelt wurden.

Der verwendete RIA bestand aus folgenden Komponenten:
- mit Anti-Osteocalcin (37-49)-Antikörper (polyklonal, Schaf) beschichteten Teströhrchen
- einem radioaktiv markierten Tracer (Analogpeptid N-Ace-tyl-¹²⁵I-Tyr-Gln-Glu-Ala-Phe-Arg-Arg-Phe-Phe-Gly-Pro-Val), der durch Zusatz von Leupeptin stabilisiert wurde (vgl. Patent DE 38 33 936)
- einer Standardreihe, welche aus humanem Knochenextrakt und humanem, osteocalcinfreiem Serum gebildet wurde Durchführung des RIA:

In die Teströhrchen werden jeweils 50 µl Standard bzw. 50 µl Patientenserum und 250 µl Tracer pipettiert. Nach einer 20-24-stündigen Inkubation bei 4°C wird durch einen Waschschritt freier von gebundenem Tracer getrennt und die im Röhrchen verbliebene Radioaktivität vermessen.

Wie aus HPLC-Untersuchungen bekannt ist, erkennt dieser RIA ausschließlich das intakte Osteocalcin.

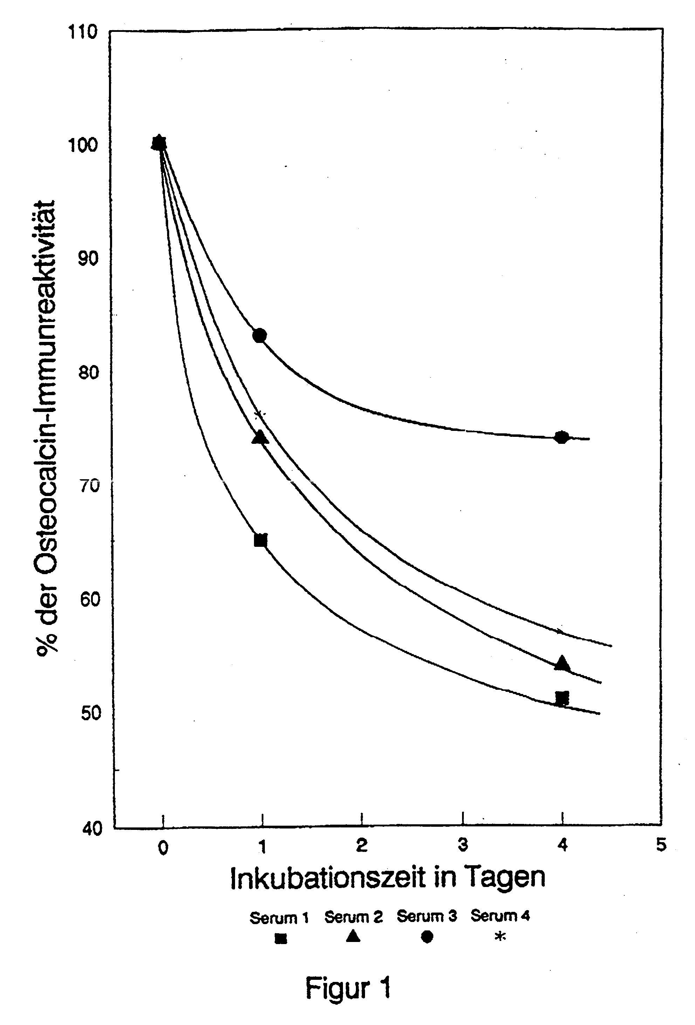

Typische Ergebnisse, wie sie infolge eines Osteocalcinabbaus in den Proben erhalten werden, sind in Fig. 1 gezeigt. Die in der Figur wiedergegebenen Ergebnisse wurden dadurch erhalten, daß man frischgewonnene Seren für die in dem Diagramm angegebenen Zeiten bei Raumtemperatur (25°C) inkubierte. Nach den angegebenen Zeiten wurde der Abbau beendet, indem die Proben eingefroren wurden. Anschließend wurden sämtliche Proben dann in einem Assaylauf mittels des oben beschriebenen RIA auf Osteocalcin vermessen.

Dabei zeigten die Kurven den Verlauf der Osteocalcin-Immunreaktivität als Maß für die Konzentration des intakten Osteocalcins in den Proben für normale Seren.

Wie Fig. 1 zu entnehmen ist, wird in allen Proben ein deutlicher Abbau des Osteocalcins (Abnahme der Osteocalcin-Immunreaktivität) erhalten, wenn Seren bei 25°C gelagert werden. Dieser Abbau läuft auch bei 4°C mit nur geringfügig verringerter Geschwindigkeit ab.

### Versuch der Hemmung des Osteocalcinabbaus durch Zusatz von Proteolysehemmstoffen

Im Einklang mit der Abnahme eines von Proteasen verursachten Osteocalcinabbaus wurde versucht, den Osteocalcinabbau durch Zusatz von Proteolysehemmstoffen (ähnlich wie in Patent DE 38 33 936) zu verhindern.

Folgende Hemmstoffe wurden auf ihre Fähigkeit hin untersucht, den in Fig. 1 gezeigten Abbau von Osteocalcin zu vermindern, wobei in den Klammern hinter den geprüften Substanzen die Inhibitorkonzentration zum Zeitpunkt der Inkubation mit Serum angeben ist:

Amastatin (100 µM), Bestatin (100 µM), Leupeptin (100 µM), Pepstatin (100 µM), Elastatinal (100 µM), Benzamidin (1 mM), Phosphoramidon (1 mM), Trasylol (1,6 x 10⁶ Einheiten/ml), Heparin (5 mg/ml), Trypsin-Inhibitor aus der Sojabohne (0,1 mg/ml), Antithrombin III (0,1 Einheiten/ml), N-Ethylmaleimid (2 mM), p-Chlormercuriphenylsulfonsäure (1 mM), Diisopropylfluorophosphat (1 mM), Phenylmethylsulfonylfluorid (2 mM), o-Phenanthrolin (5 mM), Ethylendiamintetraessigsäure (EDTA) (10 mM).

Keine dieser Substanzen führte zu einer Verringerung des Abbaus von Osteocalcin. Im Falle der Metallchelatoren EDTA und o-Phenanthrolin wurde sogar eine Beschleunigung der Osteocalcinhydrolyse beobachtet.

### Zusatz von Calciumionen (CaCl₂)

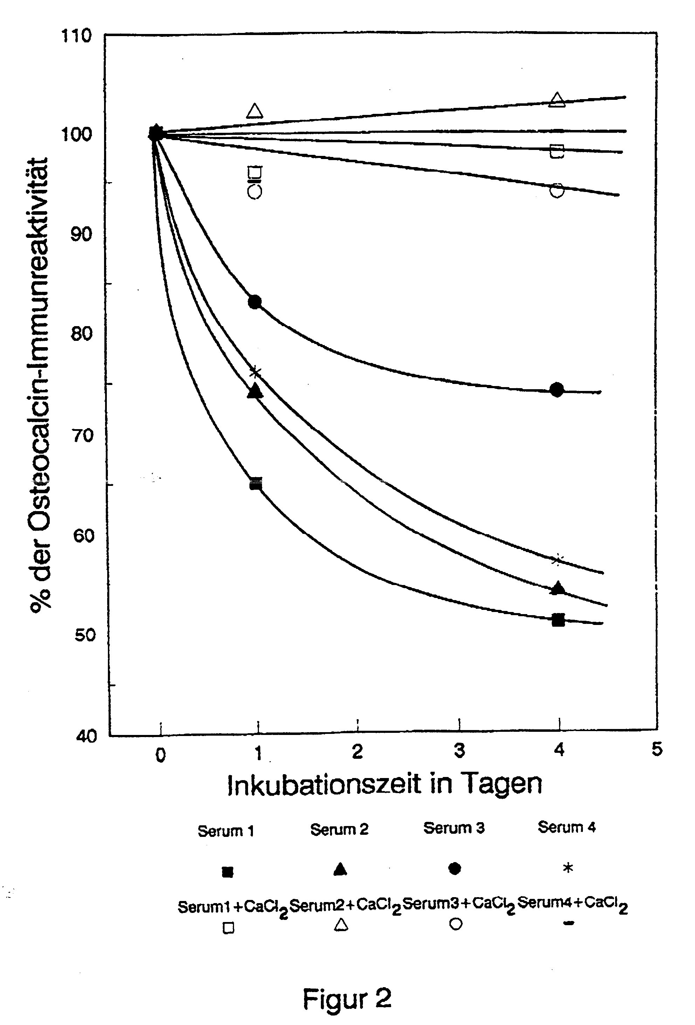

Als anstelle von Proteolysehemmstoffen CaCl₂ den Proben in einer solchen Menge zugesetzt wurde, daß die Konzentration in den Proben 20 mM betrug, wurde festgestellt, daß der Abbau von Osteocalcin vollständig unterdrückt war. Die Ergebnisse sind in Fig. 2 gezeigt.

Wie zu erkennen ist, bleibt bei Zusatz von CaCl₂ zu der Probe die Osteocalcin-Immunreaktivität über einen Zeitraum von 4 Tagen unverändert.

Um die Calciumkonzentration zu ermitteln, die als Mindestkonzentration zugesetzt werden muß, um Osteocalcin vollständig zu schützen, wurde der Effekt verschiedener CaCl₂-Konzentrationen auf den Abbau von Osteocalcin untersucht. Dazu wurden 5, 10, 20 und 40 mM zu Seren zugesetzt, die anschließend für 4 Tage bei 25°C inkubiert wurden, wonach das verbleibende Osteocalcin bestimmt wurde.

Es wurde festgestellt, daß erst oberhalb eines Zusatzes entsprechend etwa 20 mM CaCl₂ keine Veränderung der Osteocalcin-Immunreaktivität der Seren mehr nachgewiesen werden konnte. Daraus kann geschlossen werden, daß man den Seren Calciumionen in einer Konzentration von mindestens 20 mM zusetzen muß, um das endogene Osteocalcin mindestens 4 Tage bei 25°C stabil zu halten.

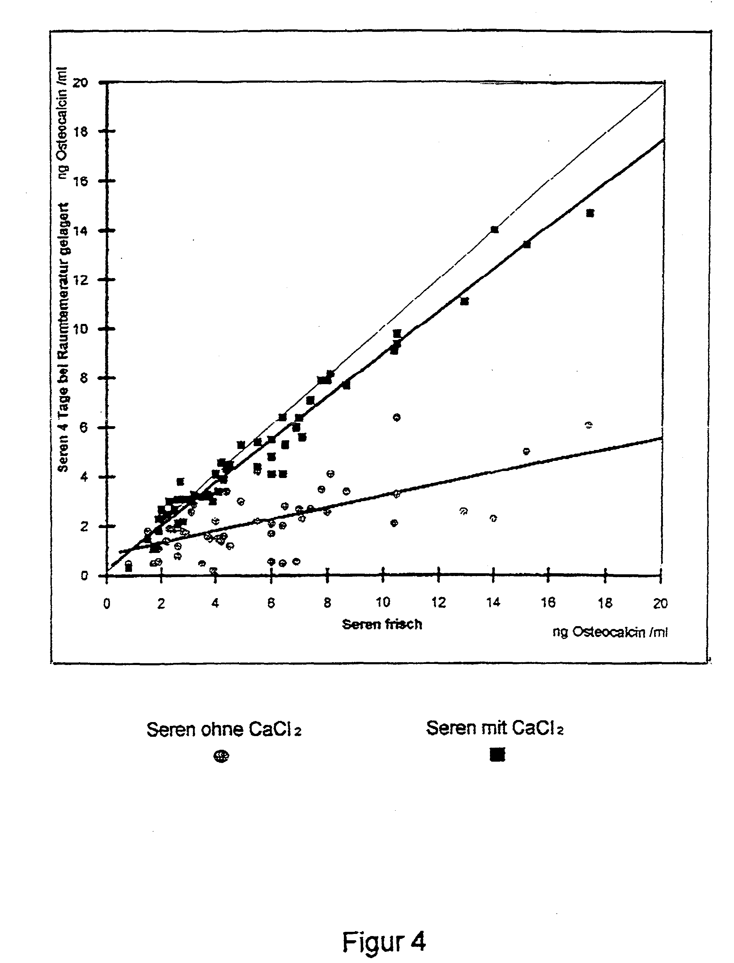

Um den Stabilisierungseffekt durch Zusatz von Calciumionen auch noch auf andere Weise zu überprüfen und dabei gleichzeitig sicherzustellen, daß es durch den stabilisiereden Zusatz von Calciumionen nicht zu Veränderungen der Immunreaktivität der Testkomponenten kommt, die die Wertigkeit der immundiagnostischen Bestimmung von Osteocalcin in Frage stellen, wurden gleiche Seren in einer Doppelbestimmung im OSCAtest BPG der Anmelderin einmal als Frischseren und zum anderen nach einer viertägigen Lagerung bei 25°C mit und ohne Zusatz von CaCl₂ bestimmt. Die Ergebnisse sind in Figur 4 dargestellt. Wie zu erkennen ist, ist die Abweichung von einer unter einem Winkel von 45° verlaufenden Geraden, die völlige Identität der Meßergebnisse für die Frischseren und die gelagerten Seren bedeuten würde, für die mit CaCl₂ stabilisierten Seren nahezu zu vernachlässigen. Die Korrelationskoeffizienten betrugen 0.98 (mit CaCl₂) bzw. 0.64 (ohne CaCl₂), die mittlere Wiederfindung betrug 94% bzw. 47%.

### Überprüfung des Stabilisierungseffekts unter Verwendung eines anderen Bestimmungsverfahrens für Osteocalcin

Um sicherzustellen, daß die oben erhaltenen Ergebnisse generelle Gültigkeit haben und nicht von dem eingesetzten konkreten RIA-Meßverfahren abhängig sind, wurde die Messung des Abbaus von Osteocalcin mittels des kommerziellen Osteocalcin RIA der Firma Isotopen Diagnostik CIS GmbH wiederholt.

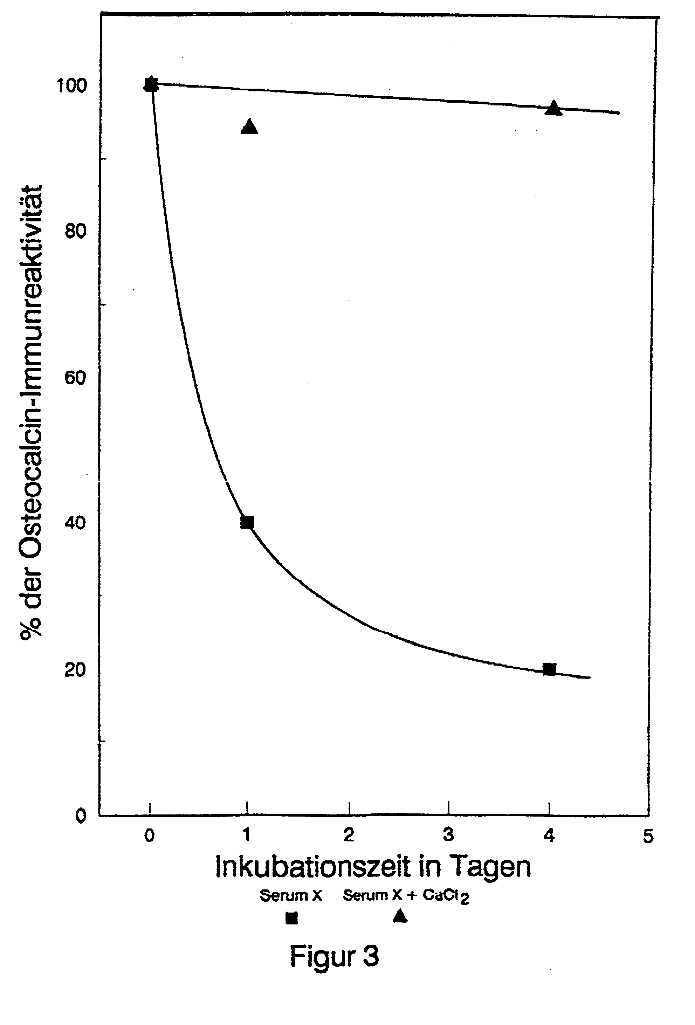

Frischgewonnene Seren wurden für die in Fig. 3 angegebenen Zeiten in An- oder Abwesenheit von CaCl₂ (20 mM) bei 25°C inkubiert. Die Bestimmung der verbleibenden Osteocalcinkonzentration mittels des angegebenen RIA zeigte, daß durch Zusatz von CaCl₂ die Abnahme der Osteocalcinkonzentration verhindert werden konnte.

Um den Abbau von Osteocalcin mit einer weiteren Methode zu untersuchen, wurde ein weiteres Experiment durchgeführt:
Radiojodiertes bovines Osteocalcin 1-49 (1 x 10⁶ cpm) wurde mit humanem Serum (300 µl) mit und ohne CaCl₂ inkubiert (48 Stunden, 25°C). Die Reaktionsmischung wurde anschließend über HPLC analysiert.

Als Trennsäule wurde eine µBondapak C₁₈-Säule der Firma Waters verwendet. Die Säule war in Laufmittel A (Wasser : Acetonitril : Trifluoressigsäure) (95 : 5 : 0,1) (V/V/V) äquilibriert. Nach dem Auftragen der Probe wurde in einem Gradienten aus Laufmittel A und Laufmittel B (Wasser : Acentonitril : Trifluoressigsäure) (10 : 90 : 0,1) (V/V/V) eluiert: In 3 min linear von 100/0 A/B nach 85/15 A/B, linear in 37 min auf 20/80 A/B und anschließend in 5 min linear auf 0/100 A/B. Die Durchflußgeschwindigkeit betrug 1 ml/min. Die Radioaktivität des Säulenausflusses wurde kontinuierlich mittels eines Ramona-Radioaktivitätsmonitors (Fa. Raytest) detektiert.

### Ergebnis:

Neben dem bovinen Osteocalcin (Elutionszeit: 17,1 min) wurden weitere Peaks (Elutionszeiten 2,4, 7,2 und 9,5 min) beobachtet, deren Entstehung jedoch durch den Zusatz von 20 mM CaCl₂ vollständig unterdrückt werden konnte.

Die Ergebnisse zeigen, daß der Abbau von bovinem Osteocalcin durch Zusatz von 20 mM CaCl₂ völlig verhindert werden kann.

## Patentansprüche

1. Verfahren zur Verhinderung des Abbaus von Osteocalcin in humanem Serums- oder Plasmaproben für eine spätere, an sich bekannte immundiagnostische Bestimmung des Osteocalcingehalts in dieser Probe, dadurch gekennzeichnet, daß man der Probe unmittelbar nach ihrer Gewinnung eine zur Verhinderung des Osteocalcinabbaus ausreichende Menge an divalenten Metallionen zusetzt.

2. Verfahren nach Anspruch 1, dadurch gekennzeichnet, daß man divalente Metallionen zusetzt, die ausgewählt sind aus Calciumionen, Magnesiumionen, Zinkionen und Cobalt (II) ionen.

3. Verfahren nach Anspruch 1 und 2, dadurch gekennzeichnet, daß man der Probe Calciumionen in einer solchen Menge zusetzt, daß die Calciumkonzentration in der Probe um mindestens 20 mM ansteigt.

4. Verfahren nach einem der Ansprüche 1 bis 3, dadurch gekennzeichnet, daß man die divalenten Metallionen der Probe in Form fester Salze oder als wäßrige Lösungen zusetzt.

5. Verfahren nach einem der Ansprüche 1 bis 4, dadurch gekennzeichnet, daß man die divalenten Metallionen dadurch der Probe zusetzt, daß man sie in den der Probengewinnung dienenden Instrumenten oder den Probengefäßen vorlegt.

6. Verfahren nach einem der Ansprüche 1 bis 5, dadurch gekennzeichnet, daß man die divalenten Metallionen in Form einer Tablette zusetzt, die eine auf das Probenvolumen abgestimmte Menge an Metallionen, insbesondere Calciumionen enthält.

7. Instrumente oder Probengefäße zur Gewinnung von Blutproben und zu deren Aufbewahrung und Verarbeitung zu Serum- oder Plasmaproben für die Durchführung des Verfahrens gemäß einem der Ansprüche 1 bis 6, dadurch gekennzeichnet, daß diese Instrumente oder Probengefäße die erforderliche Menge an divalenten Metallionen in flüssiger oder fester Form enthalten.

8. Instrumente oder Probengefäße nach Anspruch 7, dadurch gekennzeichnet, daß sie die Metallionen in Form eines in der Probe löslichen festen Films auf ihren Innenwänden enthalten.

## Claims

1. Method for preventing the degradation of osteocalcin in human serum or plasma samples for a subsequent, per se known immunodiagnostic determination of the osteocalcin content in said samples, characterized in that an amount of divalent metal ions sufficient to prevent osteocalcin degradation is added to the sample immediately after it has been obtained.

2. Method according to Claim 1, characterized in that divalent metal ions selected from the group consisting of calcium ions, magnesium ions, zinc ions and cobalt(II) ions are added.

3. Method according to Claims 1 and 2, characterized in that calcium ions are added to the sample in an amount such that the calcium concentration in the sample increases by at least 20 mM.

4. Method according to any of Claims 1 to 3, characterized in that the divalent metal ions are added to the sample in the form of solid salts or as aqueous solutions.

5. Method according to any of Claims 1 to 4, characterized in that the divalent metal ions are added to the sample by previously placing them in the instruments used for obtaining the sample or in the sample vessels.

6. Method according to any of Claims 1 to 5, characterized in that the divalent metal ions are added in the form of a tablet which contains an amount of metal ions, in particular calcium ions, adjusted to the sample volume.

7. Instruments or sample vessels for obtaining blood samples and for storing them and processing them to serum samples or plasma samples for carrying out the method according to any of Claims 1 to 6, characterized in that these instruments or sample vessels contain the required amount of divalent metal ions in liquid or solid form.

8. Instruments or sample vessels according to Claim 7, characterized in that they contain metal ions in the form of a solid film on their inner walls, which film is soluble in the sample.

## Revendications

1. Procédé pour inhiber la dégradation de l'ostéocalcine dans des échantillons de sérum ou de plasma humain en vue d'une détermination ultérieure, par un diagnostic immunologique connu, de la teneur en ostéocalcine de cet échantillon, caractérisé en ce qu'on ajoute à l'échantillon immédiatement après son prélèvement une quantité d'ions métalliques divalents suffisante pour empêcher la dégradation de l'ostéocalcine.

2. Procédé suivant la revendication 1, caractérisé en ce qu'on ajoute des ions métalliques divalents que l'on choisit parmi des ions calcium, des ions magnésium, des ions zinc et des ions cobalt (II).

3. Procédé suivant les revendications 1 et 2, caractérisé en ce qu'on ajoute à l'échantillon des ions calcium en une quantité telle que la concentration en calcium de l'échantillon s'élève d'au moins 20 mM.

4. Procédé suivant l'une des revendications 1 à 3, caractérisé en ce qu'on ajoute les ions métalliques divalents à l'échantillon sous forme de sels solides ou de solutions aqueuses.

5. Procédé suivant l'une des revendications 1 à 4, caractérisé en ce qu'on ajoute les ions métalliques divalents à l'échantillon en les disposant préalablement dans les instruments servant au prélèvement d'échantillons ou dans les récipients à échantillons.

6. Procédé suivant l'une des revendications 1 à 5, caractérisé en ce qu'on ajoute les ions métalliques divalents sous forme d'un comprimé qui contient une quantité d'ions métalliques, notamment d'ions calcium, adaptée au volume de l'échantillon.

7. Instruments ou récipients à échantillons pour le prélèvement d'échantillons de sang et pour leur conservation et leur transformation en échantillons de sérum ou de plasma pour la mise en oeuvre du procédé suivant l'une des revendications 1 à 6, caractérisés en ce que ces instruments ou récipients à échantillons contiennent la quantité nécessaire d'ions métalliques divalents sous forme liquide ou solide.

8. Instruments ou récipients à échantillons suivant la revendication 7, caractérisés en ce qu'ils contiennent les ions métalliques sous forme d'un film solide soluble dans l'échantillon sur leurs parois intérieures.
